# EUROPEAN PATENT APPLICATION

(11) **EP 4 704 089 A1**
(43) Date of publication of application: **04.03.2026**
(21) Application number: 24796746.6
(22) Date of filing: 05.04.2024
(51) Int. Cl.: G16B 45/00, G01N 27/62, G16B 10/00

(54) **ANALYSIS DEVICE, ANALYSIS SYSTEM, DISPLAY METHOD, AND PROGRAM**

(30) Priority: 26.04.2023 JP 2023072080
(71) Applicant: SHIMADZU CORPORATION, Kyoto-shi, Kyoto 604-8511 (JP); National Institute of Advanced Industrial Science and Technology, Chiyoda-ku Tokyo 100-8921 (JP)
(72) Inventor: TERAMOTO, Kanae, Kyoto-shi, Kyoto 604-8511 (JP); SEKIGUCHI, Yuji, Tsukuba-shi, Ibaraki 305-8561 (JP)
(74) Representative: Mewburn Ellis LLP
(86) International application number: PCT/JP2024/014027
(87) International publication number: WO 2024/224988

(57) **Abstract**

A controller produces a matrix (M) defined by a first axis and a second axis and representing identification results in a table format. A display displays the matrix (M). The matrix (M) contains one or more first items (L1) arranged on the first axis, a plurality of second items (L2) arranged on the second axis, as well as cells (C) each positioned corresponding to a certain first item (L1) and a certain second item (L2). The one or more first items (L1) are names of the biological samples that underwent identification. The plurality of second items (L2) are biological names arranged based on phylogenetic classification. Each cell (C) displays an identification count which is the number of times a biological sample specified by a corresponding first item (L1) was identified as a biological name specified by a corresponding second item (L2).

## Description

### TECHNICAL FIELD

The present invention relates to an analytical apparatus, an analytical system, a display method, and a program, and more specifically, it relates to a technique to display results of analysis of microorganisms.

### BACKGINSTANCE ART

Methods for identifying living things (microorganisms, for example) based on phylogenetic classification are known, such as, for example, analysis of DNA sequences and analysis of expression patterns of substances such as proteins (mass spectrometry, for example). Also, researches have been conducted on methods for displaying identification results obtained by these analysis methods.

For example, NPL 1 discloses production of a database of mass spectra of microbial samples, and for the purpose of evaluating the database, it also discloses a matrix to be used for comparing the mass spectra of the microbial samples with mass spectra in an existing database. This matrix displays numerical values that represent the degree of matching between the mass spectra of the microbial samples and the mass spectra in an existing database, and it also displays a heat map which is color-coded with colors that correspond to the respective numerical values. This makes it possible to check what is the most probable microorganism that each microbial sample of interest was identified as.

### CITATION LIST

### NON PATENT LITERATURE

NPL 1: Murugaiyan et al., "MALDI Spectra Database for Rapid Discrimination and Subtyping of Mycobacterium kansasii", Frontiers in Microbiology, Volume 9, 3 April 2018, https://doi.org/10.3389/fmicb.2018.00587
NPL 2: Lin et al., "Short-term effects of temperature on the abundance and diversity of magnetotactic cocci", Microbiology Open, Volume 1, Issue 1, pp. 53-63, March 2012, https://doi.org/10.1002/mbo3.7
NPL 3: Kiyofumi OHKUSU, New method for identifying bacteria by mass spectrometry technique, MODERN MEDIA, vol. 58, No. 4, 2012
NPL 4: "MALDI microorganism identification test (Biotyper)", [online], [retrieved on February 10, 2023] TechnoSuruga Laboratory Co., Ltd., Internet <URL:https://www.tecsrg.co.jp/services/microbiological/quality-health/maldib/>

### SUMMARY OF INVENTION

### TECHNICAL PROBLEM

With a heat map display of identification results as disclosed by NPL 1, it is possible to see what microorganisms the microbial samples of interest were identified as, but it is still not easy to check the credibility (validity) of the identification.

The present disclosure has been devised to overcome the above-described shortcoming, and it aims at enabling a user to easily assess the validity of identification results of biological samples.

### SOLUTION TO PROBLEM

An analytical apparatus according to a first aspect of the present disclosure displays identification results of one or more types of biological samples. The analytical apparatus comprises a controller and a display. The controller produces a matrix defined by a first axis and a second axis and representing the identification results in a table format. The display displays the matrix. The matrix contains one or more first items arranged on the first axis, a plurality of second items arranged on the second axis, as well as cells each positioned corresponding to a certain first item and a certain second item. The one or more first items are names of the biological samples that underwent identification. The plurality of second items are biological names arranged based on phylogenetic classification. Each cell displays an identification count which is the number of times a biological sample specified by a corresponding first item was identified as a biological name specified by a corresponding second item.

An analytical system according to a second aspect of the present disclosure displays identification results obtained by multiple rounds of identification performed for one or more types of biological samples. The analytical system comprises a memory, a processor, and a display. The memory stores the identification results. The processor produces a matrix defined by a first axis and a second axis and representing the identification results in a table format. The display displays the matrix. The processor is configured to calculate an identification count based on the identification results, where the identification count is the number of times a certain biological sample was identified as a certain biological name. The processor is also configured to arrange names of the biological samples on the first axis as one or more first items, and determine an order to arrange the biological names thus identified on the second axis as a plurality of second items, based on phylogenetic classification. A cell positioned corresponding to a certain first item and a certain second item displays an identification count which is the number of times a biological sample specified by the certain first item was identified as a biological name specified by the certain second item.

A display method according to a third aspect of the present disclosure is a method that is executed by a computer to display identification results of one or more types of biological samples, and the method comprises acquiring the identification results, and displaying a matrix defined by a first axis and a second axis and representing the identification results in a table format. The matrix contains one or more first items arranged on the first axis, a plurality of second items arranged on the second axis, as well as cells each positioned corresponding to a certain first item and a certain second item. The one or more first items are names of the biological samples that underwent identification. The plurality of second items are biological names arranged based on phylogenetic classification. The displaying includes displaying an identification count in each cell, where the identification count is the number of times a biological sample specified by a corresponding first item was identified as a biological name specified by a corresponding second item.

### ADVANTAGEOUS EFFECTS OF INVENTION

The present disclosure makes it possible for a user to easily assess the validity of identification results of biological samples.

### BRIEF DESCRIPTION OF DRAWINGS

Fig. 1 is a schematic view illustrating the configuration of an analytical system according to an embodiment.
Fig. 2 is a view for explaining a display of identification results according to an embodiment.
Fig. 3 is a view for explaining a display of a composite item.
Fig. 4 is a flowchart illustrating the outline of the process of displaying identification results according to an embodiment.
Fig. 5 is an example of a display of identification results according to an embodiment.
Fig. 6 is an example of a display of identification results according to an embodiment.

### DESCRIPTION OF EMBODIMENTS

In the following, a detailed description will be given of embodiments of the present invention, with reference to drawings. The same or corresponding portions in the drawings are denoted by the same reference characters, and basically, the description thereof is not repeated.

### [1. Configuration of Analytical Apparatus]

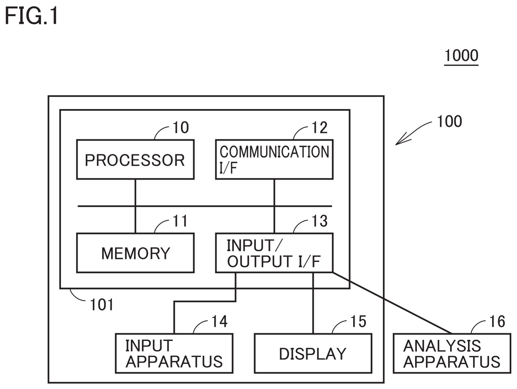

Fig. 1 is a schematic view illustrating the configuration of an analytical system 1000 according to an embodiment of the present invention.

Fig. 1 shows the configuration of analytical system 1000 according to an embodiment. In an example, analytical system 1000 displays identification results obtained by multiple rounds of identification performed for one or more types of biological samples. Analytical system 1000 includes an analytical apparatus 100 and an analysis apparatus 16. In an example, analytical apparatus 100 is an apparatus that displays identification results of biological samples analyzed by analysis apparatus 16. In an example, the biological samples include a microorganism.

Referring to Fig. 1, analytical apparatus 100 includes a controller 101, an input apparatus 14, and a display 15. Analytical apparatus 100 is typically a computer. To controller 101, input apparatus 14 and display 15 are connected. Input apparatus 14 is typically configured with a touch panel, a keyboard, a mouse, and/or the like. Input apparatus 14 receives operation inputs that a user makes to a processor 10. For example, display 15 is a display device. For example, display 15 displays an image related to receipt of an input at input apparatus 14, and displays results of processing performed by processor 10.

Controller 101 has processor 10, a memory 11, a communication interface (I/F) 12, and an input/output I/F 13, as main components. These units are communicatively connected with one another via a bus.

Processor 10 is typically an arithmetic processing unit such as a CPU (Central Processing Unit) or an MPU (Micro Processing Unit). Processor 10 controls the operation of analytical apparatus 100 by reading and executing a program stored in memory 11. The program includes a program that is executable by a computer to make the computer display the identification results of the biological samples analyzed by analysis apparatus 16.

Memory 11 is implemented by a storage apparatus such as a ROM (Read Only Memory), a RAM (Random Access Memory), and an HDD (Hard Disk Drive), for example. A ROM is capable of storing a program that is to be executed by processor 10. A RAM is capable of temporarily storing data that is to be used by processor 10 during execution of a program, and functioning as a temporary data memory used as a work area. An HDD is a non-volatile storage apparatus. In addition to an HDD or instead of an HDD, a semiconductor storage apparatus such as a flash memory may be adopted. The program and/or data may be stored in an external storage apparatus that is accessible by processor 10.

Communication I/F 12 is a communication interface for exchanging various data with an external apparatus, and is configured by an adaptor, a connector, or the like. The mode of communication may be wireless communication via wireless LAN (Local Area Network) and/or the like, or may be wired communication with the use of an USB (Universal Serial Bus) and/or the like.

Input/output I/F 13 is an interface for exchanging various data between processor 10 and an external device that is connected to input/output I/F 13. The external device includes input apparatus 14 and display 15. To input/output I/F 13, analysis apparatus 16 can be connected.

Analysis apparatus 16 is an apparatus for analyzing biological samples. For example, analysis apparatus 16 is a mass spectrometry apparatus that performs mass spectrometry of components contained in biological samples. In an example, analysis apparatus 16 is MALDI-TOF MS (Matrix-Assisted Laser Desorption/Ionization Time-of-Flight Mass Spectrometry). Analysis apparatus 16 irradiates a biological sample with a laser, makes ions thus generated enter into a flight tube, flies them to separate them from each other based on the time of flight, and then detects them. The time of flight correlates with the mass-to-charge ratio, m/z, of components contained in the biological sample. As a result, a mass spectrum is obtained which has the m/z on the horizontal axis and the detected ion intensity on the vertical axis. The mass spectrum includes peaks that correspond to the mass-to-charge ratios (m/z) of the components in the biological sample, such as proteins. Hence, by referring to the pattern of the mass spectrum, more specifically, by referring to the pattern of the peaks, it is possible to identify the proteins contained in the biological sample.

Different types of living things contain different proteins, and, accordingly, they exhibit different mass spectrum patterns. In other words, generally, the mass spectrum pattern reflects the classification of the living thing contained in the biological sample. Herein, "classification" of a living thing includes classification of the living thing based on at least one of the genotype, the strain, and the phylogenetic classification rank (level) such as subspecies, species, genus, and/or family, for example.

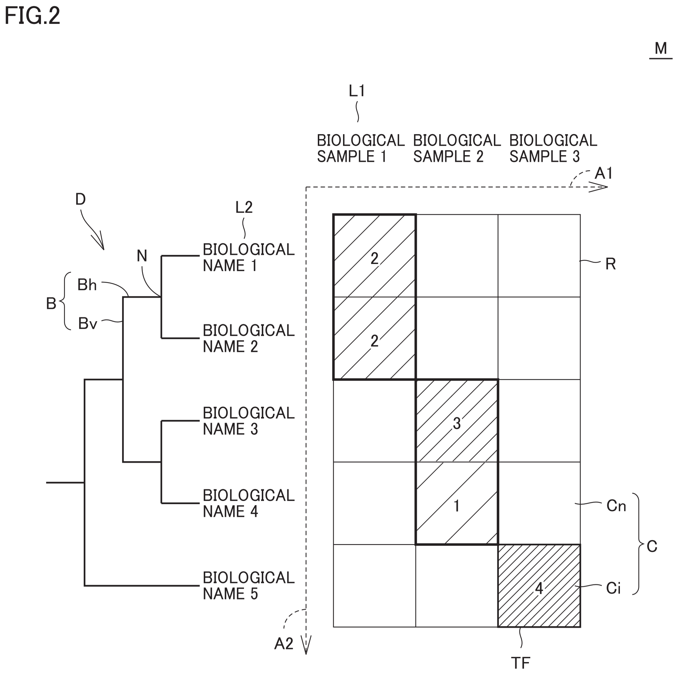

Analysis apparatus 16 sends a mass spectrum obtained by mass spectrometry, to analytical apparatus 100. Based on the mass spectrum, processor 10 identifies the living thing contained in the biological sample. For example, processor 10 identifies the living thing by using a mass spectrum database that contains mass spectra produced by calculation of theoretical m/z values of proteins based on genomic information. In an example, processor 10 finds, from the mass spectra in the mass spectrum database, a mass spectrum closest to the mass spectrum of the biological sample, and determines that the biological name corresponding to the closest mass spectrum is the name of the living thing contained in the biological sample. In other words, it identifies that the name of the living thing contained in the biological sample is the biological name corresponding to a mass spectrum closest to the mass spectrum of the biological sample. It should be noted that processor 10 may identify microorganisms by using a mass spectrum database that contains mass spectra obtained by actual measurement. Processor 10 displays a matrix (table) containing the identification results, on display 15. By visually checking the matrix, the user can easily assess the validity of the identification results. Details of the display of the identification results as well as the assessment of the validity of the identification results will be described in Fig. 2 and beyond.

Herein, "identification results" include classification of a living thing in a certain biological sample, determined based on a certain analysis result (for example, a mass spectrum) of the biological sample. In an example, "identification results" include a biological name corresponding to the biological sample determined based on the above-mentioned analysis result, for example. Moreover, "validity of identification results" refers to how correct the identification results are conceivable to be, and generally, it means a degree that is also called credibility of the identification results or reliability of the identification results.

Analysis apparatus 16 is not limited to a mass spectrometry apparatus as long as it is an apparatus that classifies biological samples based on biological characteristics. The biological characteristics are at least one of phylogenetic classification, fermentation property, type of pigment to be produced, and serotype, for example. Another example of analysis apparatus 16 is FT-IR (Fourier Transform Infrared Spectrometer).

Analytical apparatus 100 is not necessarily configured in the form of one computer, and it may be configured in the form of a plurality of computers.

### [2. Conventional Method for Displaying Identification Results of Living Things]

As a method for estimating the phylogenetic position of a biological sample, a method based on genomic information, a method based on expression patterns of proteins, a method based on other biological characteristics, and the like are known. Also, researches have been conducted on methods for displaying identification results obtained by such methods. By referring to the identification results displayed by such display methods, it is possible to obtain information about identification of the biological sample (for example, information about the most probable classification to which the biological sample can be identified), which is to be described below. However, it has not been easy to assess the validity of the identification results only by referring to the identification results thus displayed.

NPL 1 discloses a table that displays the degree of matching between mass spectra of M. kansasii of different genotypes obtained by mass spectrometry and mass spectra in an existing database. In the cells of the table, numerical values representing the degree of matching between the mass spectra of certain M. kansasii samples and mass spectra in a certain existing database are displayed, on a scale of 0 to 1. Also, the table is displayed in the form of a heat map in which the cells are color-coded depending on how great or small the respective numerical values are.

A heat map display in which all the cells are colored like in NPL 1 allows for checking what is the most probable microorganism as which a certain microbial sample was identified. However, from a heat map display in which all the cells are colored, it is not easy to assess the validity of the identification results due to, for example, the following reasons: (1) too much information is presented; and (2) much of the decision about which shades deserve attention as identification results is left to the user. Furthermore, with all the cells thus colored, recognition of the color of a certain cell may be affected by the colors of surrounding cells. To be more specific, cells with the same numerical value and the same color tend not to be recognized as the same color if they are surrounded by cells of different colors. Such optical illusion of colors makes it difficult to visually assess the validity of the identification results.

NPL 2 discloses a heat map table that displays the abundance (of genes) of different phylogenetic groups of freshwater magnetotactic cocci at different temperatures. To be specific, the vertical axis represents a dendrogram-based classification, and the horizontal axis represents the microcosms in which the freshwater magnetotactic cocci exist, at different temperatures. Each cell is color-coded to represent the proportion of the phylogenetic group of freshwater magnetotactic cocci that exists in the microcosm. Referring to this drawing disclosed by NPL 2, it is possible to understand that communities are formed in a temperaturedependent manner, and it is also possible to understand what phylogenetic classification group of freshwater magnetotactic cocci is abundant in what temperature range. However, because all the cells are colored like in the table disclosed by NPL 1, it is not easy to visually assess the validity of the communities thus discriminated.

NPL 3 discloses a table that displays results of identification of strains performed on MALDI Biotyper (Bruker), where the identification results are displayed as scores as well as symbols (ranks) and colors based on the scores. The scores represent how close the mass spectrum of a biological sample is to the strains in the database. In addition, as disclosed by NPL 3, candidate species are displayed in descending order of the score. When the score is 2.0 or more, high reliability at the species level is suggested; and when the score is not less than 1.7 and less than 2.0, a match at the genus level is suggested.

However, Biotyper has the following issues: due to variations in the microbial samples that were analyzed by mass spectrometry for database construction or due to variations in the method of pretreatment of the microbial samples, and also due to insufficient accuracy of the database, the accuracy of the identification results is not necessarily perfect. Moreover, the scores are calculated solely based on the degree of similarity between mass spectrum patterns, independent of the classification system. Therefore, usually, a plurality of probable strains including the most probable strain are obtained and when the same biological sample is analyzed multiple times, such most probable strains are sometimes different at the genus level. For this reason, the reliability of identification results obtained by Biotyper may not be necessarily high. Even when the identification results are displayed in descending order of the score as in the table disclosed by NPL 3, it is not easy to assess the validity of the identification results.

NPL 4 discloses a drawing that shows an example of identification results of commissioned Biotyper analysis that can be provided by TechnoSuruga Laboratory Co., Ltd.. To be specific, it discloses a tree-like diagram, "Cluster Analysis", produced with the use of commissioned specimens. As disclosed by NPL 4, it is possible to produce a dendrogram by using software included in Biotyper, but it is merely results of cluster analysis based on mass spectra. As discussed so far, Biotyper cluster analysis is merely a rough grouping of multiple specimens, potentially independent of phylogenetic classification based on an existing classification system and an existing evolutionary tree. From a tree-like diagram created by cluster analysis, due to a lack of a clear numerical criterion such as "When the degree of similarity is equal to or more than a certain proportion, they belong to the same species.", for example, it is impossible to determine what position in classification a specimen is at and what level in classification the match or difference is at.

For these reasons, through reference to conventional displays of identification results, it has been difficult to assess the validity of the identification results.

### [3. Displaying Identification Results According to Embodiment]

In light of these circumstances, the present embodiment provides analytical apparatus 100 capable of displaying the validity of identification results in a visually understandable manner.

Fig. 2 is a view for explaining a display of identification results according to the present embodiment. Referring to Fig. 2, a matrix M represents identification results of one or more types of biological samples, obtained by multiple rounds of analysis conducted for each biological sample, in a table format. Matrix M is defined by a first axis A1 and a second axis A2. Matrix M contains one or more first items L1 arranged on first axis A1, a plurality of second items L2 arranged on second axis A2, as well as cells C each positioned corresponding to a certain first item L1 and a certain second item L2.

Moreover, in Fig. 2, a region composed of all the cells C (in other words, a region composed of columns corresponding to first items L1 and rows corresponding to second items L2) is also called a result region R. In result region R, the identification results are displayed as described below.

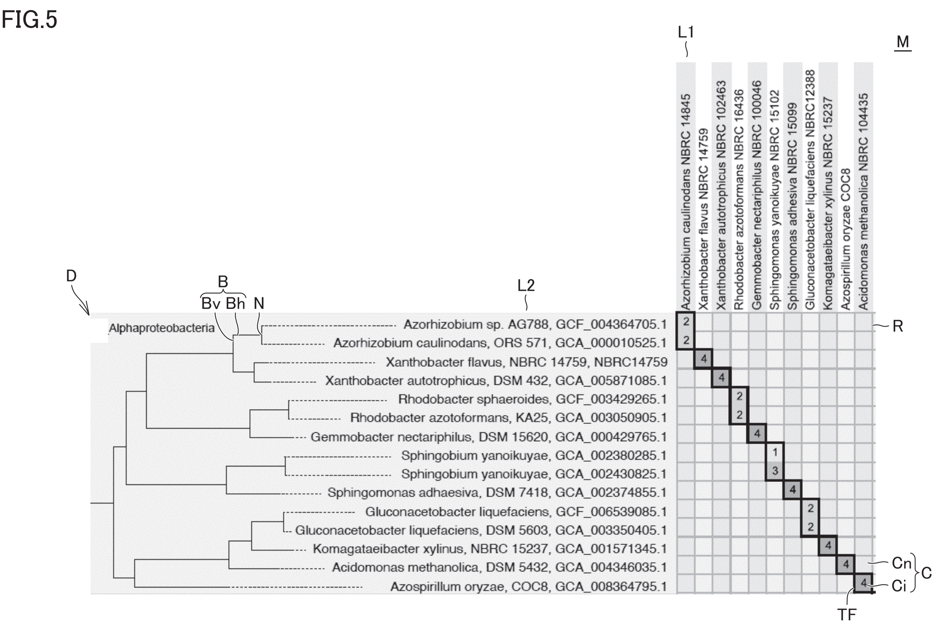

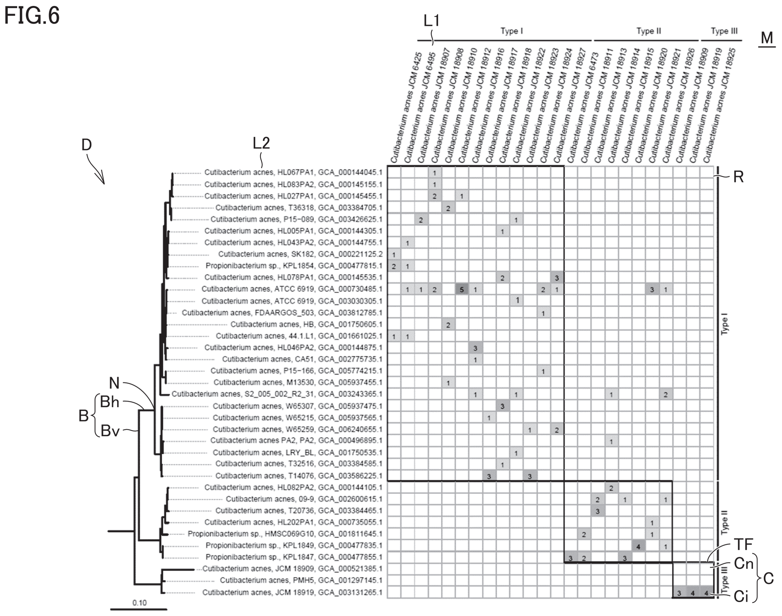

First items L1 are names of the one or more types of biological samples that underwent identification. In an example illustrated in Fig. 2, as first items L1, "BIOLOGICAL SAMPLE 1" to "BIOLOGICAL SAMPLE 3" are displayed. Regarding the style of display of the first items L1, they may be rotated upward or tilted in the columns of matrix M as illustrated in Fig. 5 and Fig. 6 for Examples 1 and 2 below.

Second items L2 are biological names arranged based on phylogenetic classification that is based on molecular phylogenetic estimation of gene sequences and the like. The expression "based on phylogenetic classification" means based on "a criterion associated with phylogenetic classification" such as the genomic base sequence, the base sequence of at least one particular gene, the base sequence for at least one particular protein, the amino acid sequence of at least one particular protein, and the like. The biological names are arranged on second axis A2 as follows: the higher the degree of similarity to each other based on "a criterion associated with phylogenetic classification", the closer to each other; and the lower the degree of similarity, the farther from each other. In an example illustrated in Fig. 2, as second items L2, "BIOLOGICAL NAME 1" to "BIOLOGICAL NAME 5" are displayed.

Each cell C displays an identification count, which is the number of times the biological sample specified by the corresponding first item L1 was identified as the biological name specified by the corresponding second item L2. In an example, each cell C displays at least one of the following: a numerical value representing the identification count, a symbol corresponding to the identification count, and a color corresponding to the identification count. With the identification count thus displayed as in these examples, it is possible to understand the identification count at a glance and/or with accuracy. In an example illustrated in Fig. 2, in a cell Ci corresponding to an identification count of 1 or more, a numerical value representing the identification count is displayed and the cell is hatched in a manner corresponding to the numerical value. On the other hand, a cell Cn corresponding to an identification count of 0 is left blank. With this, it is easily recognizable that biological sample 1 was identified twice as biological name 1, twice as biological name 2, and 0 times as the other biological names, for example. The style of displaying the identification counts in cells C is not limited to the above example, and any style may be adopted as long as it reflects the identification counts and allows for easily understanding the identification results and the validity thereof only by looking at matrix M.

Thus, in matrix M, in a cell positioned in a column corresponding to a certain type of biological sample and in a row corresponding to a biological name as which the biological sample was identified, the identification count is displayed. Such a display of identification results in matrix M is very simple, and it allows for easily recognizing what biological sample was identified as what biological name. For example, the display of identification results within result region R is highly visually understandable as compared to a display of identification results in which all the cells are colored like the table disclosed by NPL 1.

Moreover, in matrix M, because second items L2 are arranged based on phylogenetic classification, when identification results of a certain first item L1 are localized within certain bounds along second axis A2, it is conceivable that the validity is high. In other words, when identification results of a certain first item L1 are concentrated locally somewhere within matrix M, it is conceivable that the identification results are credible to some extent. In contrast, when identification results of a certain first item L1 are dispersed throughout matrix M, it is conceivable that the possibility of the identification results being valid is relatively low.

Thus, matrix M is capable of showing the validity of identification results in a visually understandable manner. Hence, the user can easily check the validity of identification results.

In particular, as the number of biological samples and identified biological names in the identification results increases, the number of displayed cells C increases, and the effect of enhanced visual understandability of the display of identification results according to the present embodiment becomes significant. For example, in the case of identification of biological samples by MALDI-TOF MS, it is often desired to consider identification results of ten to several dozen, or even more, biological samples, and therefore, the method of displaying identification results according to the present embodiment is useful (see Fig. 5 and Fig. 6 corresponding to Examples 1 and 2 described below).

Matrix M may further contain visual expression for assessment of the validity of identification results. For example, in Fig. 2, for enhancing visual understandability of the identification results, identification results of a certain biological sample are surrounded by a thick frame TF. With this, it is even easier to check the variations of identification results of the certain biological sample.

In an example, matrix M displays identification results based on mass spectra obtained by MALDI-TOF MS. This allows the user to easily understand the correlation between the MALDI-TOF MS identification results and phylogenetic classification. In particular, this allows for easily understanding the validity of the MALDI-TOF MS identification results.

In an example, the biological sample is a living thing of unknown classification. A "living thing of unknown classification" refers to a living thing the classification of which is unknown at least for the user. In this case, first item L1 is a string of characters that specifies the biological sample, for example, and an example thereof is a sample number. By referring to matrix M, it is possible to assess the validity of identification results of a living thing of unknown classification.

In an example, the biological samples include a living thing of known classification. In this case, first item L1 is the name of a living thing contained in the biological sample, for example. First items L1 may be arranged based on a biological characteristic of the living thing of known classification. In this case, not only the validity of the identification results of individual biological samples but also the relationship between the biological characteristic and phylogenetic classification can be checked. For example, it is possible to check the relationship of a certain biological characteristic with phylogenetic classification, and the relationship with variations of the identification results.

Herein, the biological characteristic is at least one of a certain "criterion associated with phylogenetic classification", the fermentation property, the type of pigment to be produced, and the serotype, for example. With this, it is possible to check if the biological characteristic is related with the certain "criterion associated with phylogenetic classification" represented by second items L2 and how it is related with the variations of the identification results. Particularly in the case where first items L1 are arranged based on a similar "criterion associated with phylogenetic classification" to the one for second items L2, if an identification count equal to or more than 1 is displayed within a region relatively close to the diagonal line that runs from the top-left corner of matrix M to the bottom-right corner as in Fig. 2, it is conceivable that the validity of the identification results of all the biological samples specified by first items L1 is high.

In an example, second items L2 are arranged based on the degree of similarity in terms of at least one of the above-mentioned "criteria associated with phylogenetic classification" (the genomic base sequence, the base sequence of at least one particular gene, the base sequence for at least one particular protein, and the amino acid sequence of at least one particular protein). In an example, the user may determine the "criterion associated with phylogenetic classification" for arranging second items L2, in accordance with the extent of diversity of classifications of the biological samples. In an example, in the case where the classifications of the biological names specified as second items L2 are relatively diverse (for example, when they are different from each other at the domain level), the user arranges second items L2 based on the amino acid sequences, which evolve relatively slowly. On the other hand, in the case where the classifications of the biological names specified as second items L2 are less diverse (for example, when they are the same at the species level), the user arranges second items L2 based on the base sequences, which evolve relatively rapidly. With this configuration, it is possible to arrange second items L2 properly in the order based on phylogenetic classification.

In an example, each of second items L2 includes at least one of genus, species, and subspecies. With this configuration, based on at least one rank selected from genus, species, and subspecies, it is possible to check the identification results and the validity thereof. More specifically, second items L2 may include a scientific name of a living thing. The scientific name may be a typical scientific name expressed with a generic name and a specific name of a living thing, or with a generic name and a specific epithet.

Second items L2 are not necessarily arranged in perfect conformity with phylogenetic classification. For example, in the case where it is desired to assess the identification results and the validity thereof at the genus level, second items L2 that specify different species belonging to the same genus do not necessarily need to be arranged based on phylogenetic classification. Moreover, in the case where the user considers that "The identification results are valid when the identification results belong to a particular level (for example, genus).", for example, second items L2 may be configured to be arranged regardless of phylogenetic classification at lower levels (such as at the species level, for example) lower than the particular level, as in the above-mentioned case.

In an example, matrix M further contains a dendrogram D specifying the phylogenetic classification of the plurality of second items L2. Dendrogram D represents the evolutionary distance between living things. Therefore, by visually checking dendrogram D, the user can visually understand the degree of genetic similarity between living things with ease. Dendrogram D includes a verticallyextending branch Bv connecting between biological names (or groups), a horizontally-extending branch Bh representing the evolutionary distance with its length, and a node N where branch Bv and branch Bh meet. Herein, branch Bh and branch Bv are collectively called branch B. By referring to dendrogram D of this type, the user can more easily understand the identification results and the validity thereof.

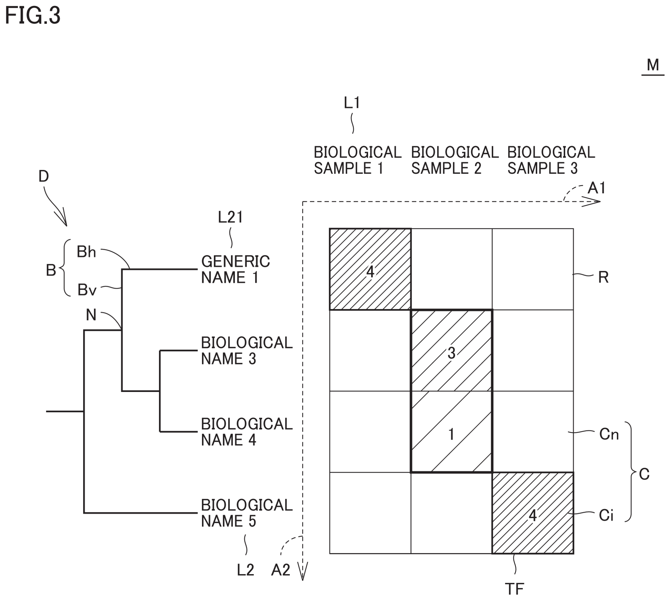

In an example, input apparatus 14 receives an input of a command to display certain adjacent second items L2 that are positioned adjacent to each other, out of all the second items L2 arranged on the second axis, in the form of one composite item L21 including contents of all the certain adjacent second items L2. Input apparatus 14 also receives an input of a command to divide the composite item L21 to display the certain adjacent second items L2. With this configuration, the user can more easily understand the extent of variations in the identification results. Take a case where biological name 1 and biological name 2 represent different species of the same genus (for example, generic name 1), as an example. In this case, if biological name 1 and biological name 2 are consolidated into composite item L21, matrix M looks like in Fig. 3. In Fig. 3, the identification results of biological sample 1 are displayed only on the row corresponding to composite item L21. From this, it is understood that biological sample 1 was analyzed four times and all these four analyses determined it as generic name 1. Accordingly, it is understood that the identification results of biological sample 1 belonged to the same classification at least at the genus level. Moreover, it is conceivable that the identification results of biological sample 1 are valid at least at the genus level. Then, by dividing the composite item L21 into the state illustrated in Fig. 2, it is possible to understand that the identification results of biological sample 1 may be different at the species level. Accordingly, it is understood that the identification results of biological sample 1 are less likely valid at the species level. In this way, by consolidating a plurality of adjacent items together and dividing composite item L21, the user can more easily understand the identification results and the validity thereof.

Preferably, the command to display certain adjacent second items L2 as composite item L21 and/or the command to divide the composite item L21 to display the certain adjacent second items L2 is input at input apparatus 14 in the form of selecting a part of dendrogram D. For example, when it is intended to consolidate biological name 1 and biological name 2 (which are second items L2) into generic name 1 (which is a composite item L21), node N connecting between biological name 1 and biological name 2 may be single-clicked to display composite item L21. For example, when it is intended to divide composite item L21 into the original two second items L2, branch Bh extending horizontally from generic name 1 may be clicked to divide composite item L21. With this configuration, the user can consolidate a plurality of adjacent items together and divide composite item L21, at a glance. This makes it even easier to understand the identification results and the validity thereof. However, it should be noted that consolidation into composite item L21 and dividing it are not limited to the above-mentioned examples; for example, biological name 1 and biological name 2 may be successively single-clicked within a certain time period to display composite item L21, and composite item L21 may be double-clicked to become divided.

In an example, when the identification count for cell C is more than a certain number, the identification count is displayed in a different expression style from when the identification count is equal to or less than the certain number. For example, the certain number is a number that not displaying the number does not affect interpretation of the identification results or the validity thereof, and/or a number that not displaying the number makes it easier to interpret the identification results and the validity thereof. An example of the certain number is 0. Another example of the certain number is a number that apparently suggests a case of an accidental error at a glance of matrix M and is therefore negligible. For example, in the case of the identification results illustrated in Fig. 2, when 100 rounds of analysis of biological sample 1 were performed and, as a result, 99 rounds identified it as biological name 1 and only 1 round identified it as biological name 5, it is possible not to display the identification result as biological name 5. This configuration enables the following, for example: a cell corresponding to an identification count more than a certain number can be displayed in an eye-catching manner as compared to a cell with equal to or less than the certain number; or a cell corresponding to an identification count equal to or less than a certain number can be displayed in a less eye-catching manner as compared to a cell with more than the certain number. As a result, it becomes easier to pay attention to the result displayed in a cell with equal to or more than the certain number. Displaying a cell in a less eye-catching manner means, for example, not displaying the numerical value representing the identification count or not color-coding the cell with a color corresponding to the identification count. For example, in Fig. 2, a cell corresponding to an identification count of 0 is left blank. This enhances the visual understandability of an identification count equal to or more than 1, and makes it even easier to understand the identification results and the validity thereof.

It should be noted that in matrix M in Fig. 2, first axis A1 is the horizontal axis and second axis A2 is the vertical axis. This configuration is more suitable for placing a horizontally-extending dendrogram D next to the second axis. Herein, a horizontally-extending dendrogram D refers to a dendrogram on which branch Bh representing the evolutionary distance extends horizontally.

It is also possible to configure first axis A1 as the vertical axis and second axis A2 as the horizontal axis; however, in this configuration, dendrogram D extends vertically. Those skilled in the art often create horizontally-extending dendrograms, and horizontally-extending dendrograms as in the example illustrated in Fig. 2 allow for easier recognition of the scientific names and the branching patterns.

Moreover, matrix M does not need to display first axis A1 and second axis A2. When they are not displayed, the display of matrix M is relatively simple, and result region R is placed closer to first items L1 and also result region R is placed closer to second items L2, which is advantageous in terms of easier recognition of the identification results and the validity thereof.

It should be noted that the style of display of matrix M is not limited to the above-mentioned examples and may vary as long as the effect of the present embodiment is exhibited; for example, instead of cells C, straight lines passing through respective first items and straight lines passing through respective second items may be displayed and the identification counts may be displayed at the points of intersection.

### [4. Process of Displaying Identification Results According to Embodiment]

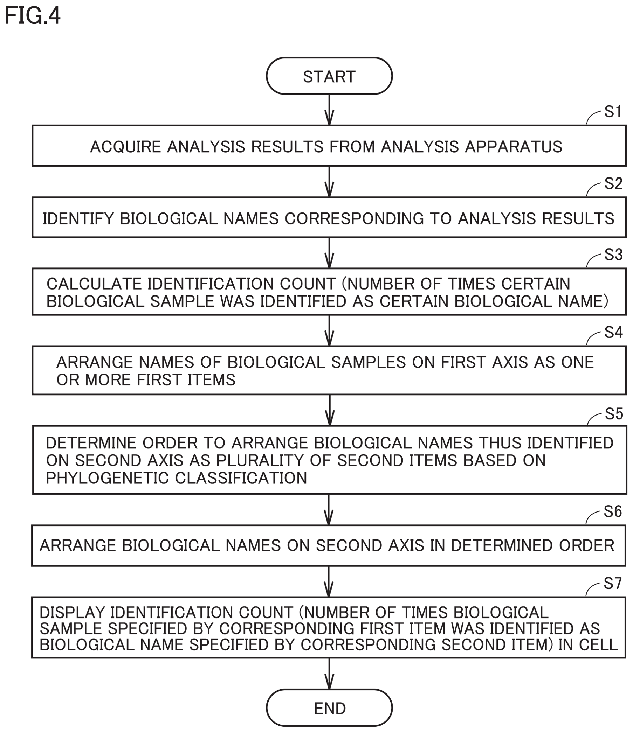

Fig. 4 is a flowchart illustrating the outline of the process of displaying identification results according to an embodiment.

In Steps 1 to 3 (hereinafter Step is expressed as "S"), processor 10 acquires identification results of one or more types of biological samples and stores them in memory 11.

In S1, processor 10 acquires a plurality of analysis results (for example, mass spectra) obtained by analysis of one or more types of biological samples each analyzed multiple times by analysis apparatus 16 (for example, MALDI-TOF MS).

In S2, processor 10 checks the analysis results against a database containing a plurality of results of analysis of living things of known classification conducted by analysis apparatus 16, to identify biological names corresponding to the analysis results. For example, to each of the mass spectra corresponding to a certain type of biological sample, processor 10 selects the closest mass spectrum from a mass spectrum database containing mass spectra of living things of known classification, and identifies a biological name corresponding to the selected mass spectrum. In an example, processor 10 selects a mass spectrum with a highest score from the mass spectrum database, where the score represents the degree of similarity. With this configuration, it is possible to obtain, from the mass spectrum database, a biological name corresponding to a mass spectrum that is closest to the mass spectrum of the identification-target biological sample, as an identification result. As needed, processor 10 may acquire, from the mass spectrum database, all the biological names that correspond to mass spectra having degree-of-similarity scores ranked equal to or higher than a certain rank, as identification results. In this way, by the process of S1 to S2, it is possible to identify biological names corresponding to the analysis results in an easy and simple manner.

In S3, processor 10 calculates an identification count, which is the number of times a certain biological sample was identified as a certain biological name. In other words, processor 10 determines categorized totals of the identification results obtained for the mass spectra of the certain type of biological sample. By taking biological sample 2 in Fig. 2 as an example, processor 10 totals the identification count for each biological name, like the identification count as biological name 3 was 2 and the identification count as biological name 4 was 2.

In the above example, processor 10 produces identification results based on analysis results acquired from analysis apparatus 16; alternatively, processor 10 may acquire identification results produced by an external apparatus and receive them via communication I/F 12.

In S4 to S7, processor 10 produces matrix M defined by first axis A1 and second axis A2 and representing the identification results, and displays it on display 15 (for example, a display device).

In S4, processor 10 arranges the names of the biological samples on first axis A1 as one or more first items L1.

In S5, processor 10 determines the order to arrange the biological names thus identified on second axis A2 as a plurality of second items L2, based on phylogenetic classification. More specifically, processor 10 calculates the degree of similarity between the biological names. Then, it arranges the biological names so that those with high degrees of similarity based on phylogenetic classification are placed close to each other and those with low degrees of similarity are placed far from each other. In an example, processor 10 arranges the biological names so that those with high degrees of similarity in genomic base sequence are placed close to each other and those with low degrees of similarity are placed far from each other.

In an example, in S5, based on the degree of similarity between the biological names, processor 10 produces dendrogram D that reflects the degree of similarity in the length of branch Bh.

In S6, processor 10 arranges the biological names on second axis A2 in the order determined in S5.

In S7, processor 10 displays an identification count in each cell C, where the identification count is the number of times the biological sample specified by the corresponding first item L1 was identified as the biological name specified by the corresponding second item L2.

In an example, processor 10 displays the names of the one or more types of biological samples thus identified, as first items L1, and displays only the biological names that were determined as identified for the one or more types of biological samples, as second items L2 in matrix M. With this configuration, the identification results and the validity thereof can be shown in a simple format. It should be noted that second items L2 may include, as needed (for example, for reference purposes), biological names other than the biological names that were determined as identified for the one or more types of biological samples.

Analytical system 1000 may include a plurality of processors, and the processes in respective Steps may be implemented by different processors. For example, identifying biological samples in S1 to S2, determining the identification counts for respective biological names in S3, and producing and displaying matrix M in S4 to S7 may be implemented by different processors.

By the process illustrated in Fig. 4, it is possible to display matrix M in which each cell displays an identification count, which is the number of times the biological sample specified by the corresponding first item L1 was identified as the biological name specified by the corresponding second item L2. In other words, it is possible to display matrix M in which identification results and the validity thereof are shown in a simple manner. Referring to matrix M, it is possible to easily assess the validity of the identification results of biological samples. In this way, by analytical apparatus 100 according to the present embodiment, it is possible to easily assess the validity of identification results of biological samples.

### [5. Comparing Identification Results Obtained by Two or More Different Types of Analysis]

When analytical apparatus 100 displays, like in the above-described manner, identification results of one matrix M that are based on identification results obtained by one type of analysis (for example, mass spectrometry), the user can easily assess the validity of the identification results by visually checking the matrix M.

In an example, analytical apparatus 100 displays two or more matrices M respectively based on the identification results from two or more types of analysis, side by side on display 15. This allows for easily comparing two or more identification results respectively based on two or more different types of analysis. For example, identification results of MALDI-TOF MS and FT-IR, which are techniques widely used for food microbial inspection and environmental microbial analysis can be displayed together in the form of matrices M. This allows for easily comparing the MALDI-TOF MS identification results and the validity thereof to the FT-IR identification results and the validity thereof. For example, it is possible to check the extent of correlation between these two analysis techniques in terms of the identification results of a certain biological species and/or the validity thereof.

### [6. Examples]

In the following, a more detailed description will be given of the display method according to the present embodiment and the effect by way of examples, but these examples are not intended to limit the scope of the display method according to the present embodiment.

### (6-1. Example 1)

Example 1 is an example in which analytical apparatus 100 displayed identification results of 11 types of biological samples derived from the class Alphaproteobacteria, in the form of matrix M.

### (6-1-1. Preparation and Mass Spectrometry of Biological Samples)

Firstly, each of sample strains derived from the class Alphaproteobacteria (strains of known species, provided by a certain institution and/or the like) was incubated. Subsequently, the incubated bacterial cells were washed in ethanol, and then dispersed in 70% formic acid aqueous solution for lysis, followed by addition of acetonitrile thereto to extract cytoplasmic components. The extraction product in an amount of 1 µL was placed dropwise onto a sample plate and dried. α-cyano-4-hydroxycinnamic acid (CHCA) solution (1 µL) was added dropwise thereto and dried to prepare sample/matrix-mixed crystals, which were measured by MALDI-MS and thereby a mass spectrum was obtained. Four biological samples were prepared for each sample strain, and accordingly, four mass spectra were obtained for each. The CHCA solution was a 50% acetonitrile aqueous solution containing 1% trifluoroacetic acid, with a CHCA concentration of 10 mg/mL.

### (6-1-2. Acquiring Identification Results by Using Analytical Apparatus)

Firstly, for each mass spectrum of a certain sample strain, detected peaks were searched in "a mass spectrum database constructed by determining theoretical values for observed masses based on genomic information". In the mass spectrum database, information (for example, m/z) of peaks of ribosomal proteins (known as biomarker proteins useful in identification of microorganisms by MALDI-MS) and the like are registered for respective strains. Values of m/z of peaks detected on a certain mass spectrum derived from the sample strain were searched through the m/z values of peaks of the mass spectra registered in the mass spectrum database, to find a mass spectrum of the highest match rate in terms of m/z of peaks of a ribosomal protein and/or the like, and thereby a strain corresponding to the mass spectrum thus found was identified for the sample strain. In this way, strains corresponding to respective mass spectra of the certain sample strain were identified; and thus, results for respective sample strains were obtained.

### (6-1-3. Display of Matrix)

Firstly, based on the database, a dendrogram containing all the strains corresponding to all the mass spectra of all the sample strains was produced. To be specific, amino acid sequences (registered in the database) of 120 types of single-copy marker genes for all the strains were acquired. Then, based on the degree of similarity between the amino acid sequences, dendrogram D containing all the strains was produced. It was configured that the strain names (second items L2) were displayed as part of dendrogram D of the strains. Moreover, as first items L1, the names of the sample strains were arranged based on the same phylogenetic classification as the one for second items L2. Moreover, cell Ci corresponding to an identification count of the sample strain equal to or more than 1 was configured to display the numerical value representing the identification count as well as a color corresponding to the numerical value. Cell Cn corresponding to an identification count of the sample strain of 0 was configured to be left blank. It was configured that first axis A1 and second axis A2 displayed in Fig. 2 were not displayed for the purpose of enhancing visual understandability of matrix M. Matrix M thus configured was displayed on display 15 (Fig. 5).

### (6-1-4. Effect of Display of Matrix)

The sample strains analyzed in Example 1 included those different at the genus level, and therefore, it is impossible to understand the phylogenetic relationship between them only from the scientific names and without the knowledge about the phylogeny of the sample strains. On the other hand, matrix M displays dendrogram D based on genomic information as well as the distribution of the identification results of respective sample strains, in an easy-to-understand style. Therefore, even a user who does not know the phylogeny of the sample strains can easily understand which classification group the identification results belong to. Thus, a display method that allows for easily understanding the identification results of biological samples, the validity, and the tolerance level was provided.

For example, with a brief overview of matrix M, it is possible to understand a rough tendency of the identification results of respective sample strains and the validity thereof. In matrix M in Fig. 5, it is easy to understand that identification counts for each sample strain were localized at one biological name or two adjacent biological names. From this, it is conceivable that the identification results of the sample strains are mostly valid. Moreover, because the identification counts are displayed within a region relatively close to the diagonal line that runs from the top-left corner of matrix M to the bottom-right corner, it is conceivable that the validity of the identification results of all the sample strains is high.

Then, by referring to the items in matrix M, it is possible to accurately understand the identification results of respective sample strains and the validity thereof. For example, as for the identification results of the sample strain "Azorhizobium caulinnodans NBRC 14845" specified by first item L1 positioned at the left end, it is understood that the sample strain was identified twice as the strain "Azorhizobium sp. AG788, GCF_004364705.1" specified by second items L2 positioned at the top, it was identified twice as the strain "Azorhizobium caulinnodans, ORS571, GCA_000010525.1" specified by second items L2 positioned second from the top, and it was identified 0 times as the other biological names. Therefore, it is understood that the strain NBRC 14845 was at least identified as the genus Azorhizobium.

Similarly, as for the sample strain "Rhodobacter azotoformans NBRC16436" specified by first items L1 positioned fourth from the left, it is understood that the identification results may be different at the species level but they are correct at the genus level.

### (6-1-5. Display of Dendrogram and Effect Thereof)

In matrix M, with a single click of a phylogenetic classification group on dendrogram D, for example, it is possible to easily open or close the phylogenetic classification group. To be specific, in matrix M, by opening a certain classification group, it is possible to display subordinate phylogenetic groups subordinate to the classification rank, and by clicking and opening each subordinate phylogenetic group, it is possible to display the details of the subordinate phylogenetic group, all the way to the strain level on the dendrogram. Moreover, by clicking the branch B on the dendrogram again, it is possible to close the once-opened classification group, to thereby consolidate many strain-level items into branch B representing one species or consolidate many species into branch B representing one genus-level classification group. This function has made it possible to provide a display method that allows for more easily understanding what position in classification on dendrogram D a hit entry is located. Herein, a hit entry refers to a certain identified strain or all the identified strains.

Moreover, it is also possible to display the color, line width, line shape, and the like of branch B connected to a hit entry, differently from those of branch B not connected to a hit entry. This makes it even easier to understand what position in classification on dendrogram D the hit entry is located.

Thus, by using dendrogram D, the user can easily understand the identification results and the validity thereof.

### (6-2. Example 2)

Example 2 is an example in which analytical apparatus 100 displayed identification results of Cutibavterium acnes, which is known to be classified into Types I, II, III, in the form of matrix M.

### (6-2-1. Preparation and Mass Spectrometry of Biological Samples)

Firstly, the bacterial cells were dispersed in distilled water and disrupted at 4000 rpm for 3 minutes with the use of zirconia beads, and thereby a disrupted bacterial cell liquid was obtained. The disrupted bacterial cell liquid was centrifuged at 15000 g for 5 minutes, and then a tube-type ultrafiltration unit (Amicon Ultra Series; Nominal Molecular Weight Limit (NMWL) 300 kDa) was used for concentrating the ribosomal fraction (14000 g, 10 minutes). A matrix solution was prepared by dissolving sinapic acid in 50% acetonitrile aqueous solution containing 1% trifluoroacetic acid, in a concentration of 10 mg/mL. The matrix solution (9 µL) and the concentrated ribosomal fraction (1 µL) were mixed well in a microtube, and 1 µL of the mixture was placed dropwise onto a sample plate and dried to prepare sample/matrix-mixed crystals, which were subjected to MALDI-MS measurement.

### (6-2-2. Acquiring Identification Results by Using Analytical Apparatus)

Acquisition of identification results in Example 2 was carried out in the same manner as in acquisition of identification results in Example 1.

### (6-2-3. Display of Matrix)

Display of matrix M in Example 2 (Fig. 6) was carried out in the same manner as in the display of matrix M in Example 1. It should be noted that in Example 2, dendrogram D was produced based on the amino acid sequences of translation products (proteins) estimated from single-copy genes in respective microbial genomes. In Fig. 6, the thick frame TF represents a region in which the sample strain and the dendrogram D both belong to the same Type (in other words, Type I, II, or III).

### (6-2-4. Effect of Display of Matrix and Effect of Display of Dendrogram)

Cutibacteroum ances (hereinafter, C. acnes) used in Example 2 is one species, but it is known to be classified into three subtypes. Referring to Fig. 6, all the sample strains were correctly identified as C. acnes, and therefore it is considered that the identification was 100% correct at the species level. It is also understood that the sample strains of Type I and the sample strains of Type III were correctly identified to the subtype level (see the thick frame TF). Most of the sample strains of Type II were correctly identified as Type II but some of them were identified as Type I, indicating incorrect identification at the subtype level. However, referring to the dendrogram, Type I is closer to Type II than to Type III; therefore, even though the identification results were incorrect at the subtype level, it is understood that the results were correct as compared to identification as Type III.

By the way, C. acnes was called Propionibacterium acnes until several years ago. In matrix M of Fig. 6, some of the sample strains were identified as Propionibacterium sp., and these identification results can look incorrect at the genus level. However, as is clear from the dendrogram, Propionibacterium sp is close to C. acnes, and therefore it is understood that the above-mentioned identification results are not incorrect. Thus, even if the scientific name was changed, reference to the phylogenetic credibility indicates that the identification results are not incorrect.

In matrix M in Fig. 6, dendrogram D and second items L2 display the entries of strains corresponding to C. acnes, classified as the species. It should be noted that matrix M contains information about the dendrogram at higher levels than the species level and it can display the information. For example, matrix M contains information about the dendrogram at the division level and can display dendrogram D at the division level (not shown). When matrix M displays the dendrogram at the division level, the display (for example, second item L2 or branch B) corresponding to the division to which a species hit by a sample strain belongs to is displayed in a different color. Thus, even if a C. acnes sample strain is also incorrectly determined as another division, such incorrect determination can be easily found. In such a case, it can be easily understood that the validity of the identification results is low.

As shown in the above examples, it is easily understandable that with dendrogram D in matrix M modified to display more specific classification groups, the smaller the variations in the identification results, the more reliable the results can be.

### [7. Significance of Combined Display of MALDI Results and Phylogenetic Classification]

As described above, identification of biological samples by MALDI is performed based on the degree of similarity between mass spectra. Generally, the MALDI identification results reflecting the degree of similarity are displayed solely based on the degree of similarity between mass spectra as in the drawings in NPLs 3, 4. As a consequence, it is sometimes difficult to understand the relationship between the MALDI identification results and phylogenetic classification and also to determine the extent of validity. However, not only in MALDI but rather in any identification of living things in general, it is important to understand the relationship between identification results and phylogenetic classification and also to understand the extent of validity of the identification results,

With the use of analytical apparatus 100 according to the present embodiment, only by inputting identification results into analytical apparatus 100, the user can view matrix M that displays the correlation between MALDI analysis results and phylogenetic classification in a simple manner. This allows the user to understand the relationship between the identification results and phylogenetic classification as well as the validity of the identification results, with ease at a glance. In addition, by referring to dendrogram D which allows for understanding phylogenetic classification of living things at a glance, the user can better understand the relationship between the identification results and phylogenetic classification as well as the validity of the identification results.

As described above, it is conceivable that analytical apparatus 100 according to the present embodiment is particularly useful also because it enables easy assessment of the extent of validity of MALDI analysis results of biological samples.

### [Aspects]

As will be appreciated by those skilled in the art, the above-described example embodiments are specific examples of the below aspects.

(Item 1) An analytical apparatus according to an aspect displays identification results of one or more types of biological samples. The analytical apparatus comprises a controller and a display. The controller produces a matrix defined by a first axis and a second axis and representing the identification results in a table format. The display displays the matrix. The matrix contains one or more first items arranged on the first axis, a plurality of second items arranged on the second axis, as well as cells each positioned corresponding to a certain first item and a certain second item. The one or more first items are names of the biological samples that underwent identification. The plurality of second items are biological names arranged based on phylogenetic classification. Each cell displays an identification count which is the number of times a biological sample specified by a corresponding first item was identified as a biological name specified by a corresponding second item.

With the matrix displayed on the analytical apparatus according to Item 1, it is possible to show what biological sample was identified as what biological name as well as the validity of the identification results, in a visually understandable manner. This makes it possible for the user to easily assess the validity of the identification results of the biological samples.

(Item 2) In the analytical apparatus according to Item 1, the matrix further contains a dendrogram specifying the phylogenetic classification of the plurality of second items.

With the analytical apparatus according to Item 2, by referring to the dendrogram, the user can more easily understand the identification results and the validity thereof.

(Item 3) In the analytical apparatus according to Item 1 or Item 2, each of the plurality of second items includes at least one of genus, species, and subspecies.

With the analytical apparatus according to Item 3, based on at least one rank selected from genus, species, and subspecies, it is possible to check the identification results and the validity thereof.

(Item 4) In the analytical apparatus according to any one of Item 1 to Item 3, the plurality of second items are arranged based on a degree of similarity, and the degree of similarity is a degree of similarity in terms of at least one of genomic base sequence, base sequence of at least one particular gene, base sequence for at least one particular protein, and amino acid sequence of at least one particular protein.

With the analytical apparatus according to Item 4, it is possible to arrange the second items properly in the order based on phylogenetic classification.

(Item 5) The analytical apparatus according to any one of Item 1 to Item 4 further comprises an input apparatus at which a user inputs a command. The input apparatus receives: an input of a command to display certain adjacent second items that are positioned adjacent to each other, out of all the second items arranged on the second axis, in a form of one composite item including contents of all the certain adjacent second items; and an input of a command to divide the composite item to display the certain adjacent second items.

With the analytical apparatus according to Item 5, by consolidating a plurality of adjacent items together and dividing a composite item, the user can more easily understand the identification results and the validity thereof.

(Item 6) The analytical apparatus according to Item 2 further comprises an input apparatus at which a user inputs a command. The input apparatus receives: an input of a command to display certain adjacent second items that are positioned adjacent to each other, out of all the second items arranged on the second axis, in a form of one composite item including contents of all the certain adjacent second items; and an input of a command to divide the composite item to display the certain adjacent second items. The command to display the certain adjacent second items in a form of one composite item and/or the command to divide the composite item to display the certain adjacent second items is input at the input apparatus in a form of selecting a part of the dendrogram.

With the analytical apparatus according to Item 6, the user can consolidate a plurality of adjacent items together and divide a composite item, at a glance. This makes it even easier to understand the identification results and the validity thereof.

(Item 7) In the analytical apparatus according to any one of Item 1 to Item 6, the one or more types of biological samples include a living thing of unknown classification.

With the analytical apparatus according to Item 7, by referring to the matrix, it is possible to assess the validity of identification results of a living thing of unknown classification.

(Item 8) In the analytical apparatus according to any one of Item 1 to Item 7, the one or more types of biological samples include a living thing of known classification. The one or more first items are arranged based on a biological characteristic of the living thing of known classification.

With the analytical apparatus according to Item 8, not only the validity of the identification results of individual biological samples but also the relationship between the biological characteristic and phylogenetic classification can be checked.

(Item 9) In the analytical apparatus according to Item 8, the biological characteristic is at least one of phylogenetic classification, fermentation property, type of pigment to be produced, and serotype.

With the analytical apparatus according to Item 9, it is possible to check if the biological characteristic is related with a certain "criterion associated with phylogenetic classification" represented by the second items and how it is related with the variations of the identification results. Especially in the case where the first items are arranged based on a similar "criterion associated with phylogenetic classification" to the one for the second items, if an identification count equal to or more than 1 is displayed within a region relatively close to the diagonal line that runs from the top-left corner of the matrix to the bottom-right corner, it is conceivable that the validity of the identification results of all the biological samples specified by the first items is high.

(Item 10) In the analytical apparatus according to any one of Item 1 to Item 9, each cell displays at least one of a numerical value representing the identification count, a symbol corresponding to the identification count, and a color corresponding to the identification count.

With the analytical apparatus according to Item 10, with the identification count thus displayed as in these examples, it is possible to understand the identification count at a glance and/or with accuracy.

(Item 11) In the analytical apparatus according to any one of Item 1 to Item 10, when the identification count for the cell is more than a certain number, the identification count is displayed in a different expression style from when the identification count is equal to or less than the certain number.

With the analytical apparatus according to Item 11, it is easier to pay attention to the result displayed in a cell with equal to or more than the certain number.

(Item 12) In the analytical apparatus according to Item 11, when the identification count for the cell is equal to or less than the certain number, the cell is left blank.

With the analytical apparatus according to Item 12, visual understandability of an identification count equal to or more than 1 is enhanced, making it even easier to understand the identification results and the validity thereof.

(Item 13) In the analytical apparatus according to any one of Item 1 to Item 12, the one or more types of biological samples include a microorganism.

With the analytical apparatus according to Item 13, the user can easily understand the identification results of microorganisms of unknown classification, for example, and the validity thereof. As for microorganisms of known classification, not only the validity of the identification results of individual microorganisms but also the relationship between the biological characteristic and phylogenetic classification can be checked.

(Item 14) In the analytical apparatus according to any one of Item 1 to Item 13, the identification results are identification results based on mass spectra obtained by matrix-assisted laser desorption/ionization time-of-flight mass spectrometry (MALDI-TOF MS).

With the analytical apparatus according to Item 14, it is possible to easily understand the correlation between the MALDI-TOF MS identification results and phylogenetic classification. In particular, it is possible to easily assess the extent of validity of MALDI-MS analysis results of a biological sample.

(Item 15) In the analytical apparatus according to any one of Item 1 to Item 14, the analytical apparatus displays the matrix on the display, the matrix comprises two or more matrices that are based on the identification results from two or more types of analysis, respectively, and the two or more matrices are displayed side by side.

With the analytical apparatus according to Item 15, it is possible to check the extent of correlation between two or more types of analysis techniques in terms of the identification results of a certain biological species and/or the validity thereof.

(Item 16) An analytical system according to an aspect displays identification results obtained by multiple rounds of identification performed for one or more types of biological samples. The analytical system comprises a memory, a processor, and a display. The memory stores the identification results. The processor produces a matrix defined by a first axis and a second axis and representing the identification results in a table format. The display displays the matrix. The processor is configured to calculate an identification count based on the identification results, where the identification count is the number of times a certain biological sample was identified as a certain biological name. The processor is further configured to arrange names of the one or more types of biological samples on the first axis as one or more first items, and determine an order to arrange the biological names thus identified on the second axis as a plurality of second items, based on phylogenetic classification. A cell positioned corresponding to a certain first item and a certain second item displays an identification count which is the number of times a biological sample specified by the certain first item was identified as a biological name specified by the certain second item.

With the analytical system according to Item 16, it is possible to display matrix M that shows, in a visually understandable manner, what biological sample was identified as what biological name as well as the validity of the identification results. This makes it possible for the user to easily assess the validity of the identification results of the biological samples.

(Item 17) In the analytical system according to Item 16, the processor is configured to acquire analysis results obtained by analysis of the one or more types of biological samples conducted by an analysis apparatus. The processor is also configured to check the analysis results against a database containing a plurality of results of analysis of living things of known classification conducted by the analysis apparatus, to identify biological names corresponding to the analysis results.

With the analytical system according to Item 17, it is possible to identify biological names corresponding to the analysis results, in an easy and simple manner.

(Item 18) A display method according to an aspect is a method that is executed by a computer to display identification results of one or more types of biological samples, and the method comprises: acquiring the identification results; and displaying a matrix defined by a first axis and a second axis and representing the identification results in a table format. The matrix contains one or more first items arranged on the first axis, a plurality of second items arranged on the second axis, as well as cells each positioned corresponding to a certain first item and a certain second item. The one or more first items are names of the biological samples that underwent identification. The plurality of second items are biological names arranged based on phylogenetic classification. The displaying includes displaying an identification count in each cell, where the identification count is the number of times a biological sample specified by a corresponding first item was identified as a biological name specified by a corresponding second item.

With the matrix thus displayed by the display method according to Item 18, it is possible to show what biological sample was identified as what biological name as well as the validity of the identification results, in a visually understandable manner. This makes it possible for the user to easily assess the validity of the identification results of the biological samples.

(Item 19) A program that is executed by a computer to make the computer execute the display method according to Item 18.

It should be construed that embodiments disclosed herein are given by way of illustration in all respects, not by way of limitation. It is intended that the scope of the present invention is defined by claims, not by the above description, and encompasses all modifications and variations equivalent in meaning and scope to the claims.

### REFERENCE SIGNS LIST

10 processor, 11 memory, 12 communication I/F, 13 input/output I/F, 14 input apparatus, 15 display, 16 analysis apparatus, 100 analytical apparatus, 101 controller, A1 first axis, A2 second axis, B, Bh, Bv branch, C, Ci, Cn cell, D dendrogram, L1 first item, L2 second item, L21 composite item, M matrix, N node, R result region, S thick frame, T result table.

## Claims

1. An analytical apparatus that displays identification results of one or more types of biological samples, the analytical apparatus comprising:
a controller that produces a matrix defined by a first axis and a second axis and representing the identification results in a table format; and
a display that displays the matrix, wherein
the matrix contains one or more first items arranged on the first axis, a plurality of second items arranged on the second axis, as well as cells each positioned corresponding to a certain first item and a certain second item,
the one or more first items are names of the biological samples that underwent identification,
the plurality of second items are biological names arranged based on phylogenetic classification, and
each cell displays an identification count which is the number of times a biological sample specified by a corresponding first item was identified as a biological name specified by a corresponding second item.

2. The analytical apparatus according to claim 1, wherein the matrix further contains a dendrogram specifying the phylogenetic classification of the plurality of second items.

3. The analytical apparatus according to claim 1 or 2, wherein each of the plurality of second items includes at least one of genus, species, and subspecies.

4. The analytical apparatus according to claim 1 or 2, wherein the plurality of second items are arranged based on a degree of similarity, and the degree of similarity is a degree of similarity in terms of at least one of genomic base sequence, base sequence of at least one particular gene, base sequence for at least one particular protein, and amino acid sequence of at least one particular protein.

5. The analytical apparatus according to claim 4, further comprising an input apparatus at which a user inputs a command, wherein
the input apparatus receives:
an input of a command to display certain adjacent second items that are positioned adjacent to each other, out of all the second items arranged on the second axis, in a form of one composite item including contents of all the certain adjacent second items; and
an input of a command to divide the composite item to display the certain adjacent second items.

6. The analytical apparatus according to claim 2, further comprising an input apparatus at which a user inputs a command, wherein
the input apparatus receives:
an input of a command to display certain adjacent second items that are positioned adjacent to each other, out of all the second items arranged on the second axis, in a form of one composite item including contents of all the certain adjacent second items; and
an input of a command to divide the composite item to display the certain adjacent second items, and
the command to display the certain adjacent second items in a form of one composite item and/or the command to divide the composite item to display the certain adjacent second items is input at the input apparatus in a form of selecting a part of the dendrogram.

7. The analytical apparatus according to claim 1 or 2, wherein the one or more types of biological samples include a living thing of unknown classification.

8. The analytical apparatus according to claim 1 or 2, wherein
the one or more types of biological samples include a living thing of known classification, and
the one or more first items are arranged based on a biological characteristic of the living thing of known classification.

9. The analytical apparatus according to claim 8, wherein the biological characteristic is at least one of phylogenetic classification, fermentation property, type of pigment to be produced, and serotype.

10. The analytical apparatus according to claim 1 or 2, wherein each cell displays at least one of a numerical value representing the identification count, a symbol corresponding to the identification count, and a color corresponding to the identification count.

11. The analytical apparatus according to claim 1 or 2, wherein when the identification count for the cell is more than a certain number, the identification count is displayed in a different expression style from when the identification count is equal to or less than the certain number.

12. The analytical apparatus according to claim 11, wherein when the identification count for the cell is equal to or less than the certain number, the cell is left blank.

13. The analytical apparatus according to claim 1 or 2, wherein the one or more types of biological samples include a microorganism.

14. The analytical apparatus according to claim 1 or 2, wherein the identification results are identification results based on mass spectra obtained by matrix-assisted laser desorption/ionization time-of-flight mass spectrometry (MALDI-TOF MS).

15. The analytical apparatus according to claim 1 or 2, wherein the analytical apparatus is configured to display the matrix on the display, the matrix comprises two or more matrices that are based on the identification results from two or more types of analysis, respectively, and the two or more matrices are displayed side by side.

16. An analytical system that displays identification results obtained by multiple rounds of identification performed for one or more types of biological samples, the analytical system comprising:
a memory that stores the identification results;
a processor that produces a matrix defined by a first axis and a second axis and representing the identification results in a table format; and
a display that displays the matrix, wherein
the processor is configured to:
calculate an identification count based on the identification results, the identification count being the number of times a certain biological sample was identified as a certain biological name;
arrange names of the one or more types of biological samples on the first axis as one or more first items; and
determine an order to arrange the biological names thus identified on the second axis as a plurality of second items, based on phylogenetic classification, and
a cell positioned corresponding to a certain first item and a certain second item displays an identification count which is the number of times a biological sample specified by the certain first item was identified as a biological name specified by the certain second item.

17. The analytical system according to claim 16, wherein
the processor is configured to:
acquire analysis results obtained by analysis of the one or more types of biological samples conducted by an analysis apparatus; and
check the analysis results against a database containing a plurality of results of analysis of living things of known classification conducted by the analysis apparatus, to identify biological names corresponding to the analysis results.

18. A method that is executed by a computer to display identification results of one or more types of biological samples, the method comprising:
acquiring the identification results; and
displaying a matrix defined by a first axis and a second axis and representing the identification results in a table format, wherein
the matrix contains one or more first items arranged on the first axis, a plurality of second items arranged on the second axis, as well as cells each positioned corresponding to a certain first item and a certain second item,
the one or more first items are names of the biological samples that underwent identification,
the plurality of second items are biological names arranged based on phylogenetic classification, and
the displaying includes displaying an identification count in each cell, the identification count being the number of times a biological sample specified by a corresponding first item was identified as a biological name specified by a corresponding second item.

19. A program that is executed by a computer to make the computer execute the method according to claim 18.
